# EUROPEAN PATENT APPLICATION

(11) **EP 4 603 061 A1**
(43) Date of publication of application: **20.08.2025**
(21) Application number: 24158302.0
(22) Date of filing: 19.02.2024
(51) Int. Cl.: A61F 2/24

(54) **SUTURELESS TRANSCATHETER HEART VALVE PROSTHESIS**

(71) Applicant: Tresquare Technologies S.r.l., 10121 Torino (IT)
(72) Inventor: PASQUINO, Enrico, 1073 Savigny (CH); PASQUINO, Stefano, 1033 Cheseaux-sur-Lausanne (CH); OSTA, Franco, 15020 Mombello Monferrato (AL) (IT); FERRARO, Mauro, 10138 Torino (IT); OSTA, Stefano, 10015 Ivrea (IT); BARBERO, Andrea, 10048 Vinovo (IT)
(74) Representative: Grosfillier, Philippe

(57) **Abstract**

Sutureless transcatheter heart valve prosthesis comprising a collapsible stent structure with anchoring elements and a valve component located within said stent structure; characterized by the fact that the stent structure is configured to be positioned within a heart valve annulus after full excision of the native valve leaflets (1); the said stent structure comprising a sub-annular anchoring element (13), a supra-annular anchoring element (12), and wherein said sub- and supra-annular anchoring elements (13,12)are configured to cinch ridges (9) that are formed by the valve commissures (2) and the resection profile (5) after the full excision of said native valve leaflets (1).

## Description

### Field of invention

This invention relates to sutureless transcatheter heart valve prostheses, in particular aortic valve prosthesis, suitable to be implanted within the annulus of the native valve.

### Background

The replacement of stenotic native aortic valve has been surgically performed in the last 50 years. This replacement procedure requires an open-heart access with an extracorporeal circulatory bypass. The calcific aortic valve is surgically excised and replaced by a prosthetic valve that is anchored to the aortic root by stitches.

In the last years a new replacement procedure for the aortic valve has been introduced in clinical use. This procedure, called TAVI (Transcatheter Aortic Valve Implant) is based on a transcatheter approach without cardiopulmonary bypass. These new heart valve bioprostheses are collapsible and are loaded into a delivery catheter to be positioned into the heart over the diseased aortic valve. These bioprostheses are retained in place by the radial compression exerted on the calcific native valve therefore no sutures are required. These expandable heart valves are currently manufactured by self-expandable materials such as Nitinol, compressed into a valve deployment device and released on calcified native valve. Other transcatheter heart valves are balloon expandable and manufactured using stainless steel or nickel-cobalt-chromium stents.

Leaflets of TAVI (Transcatheter Aortic Valve Implant) are typically made from cross-linked tissues of animal origin, such as bovine or porcine pericardium, are stitched to the stent typically by means of stitching the leaflets onto the stent frame, normally along a pericardium or fabric skirt, or by means of direct stitching to the stent frame in the case of xenograft valves. Polymeric leaflets have also been proposed which may be attached to the stent by means of stitching, gluing or other means.

In recent years, polymeric leaflets have been part of several research programs in the attempt to replace bovine and porcine pericardium leaflets. However, these new materials are not immune from potential drawbacks. Polymeric leaflet designs causing overly close coaptation of the leaflets may limit the wash-out of blood during hemodynamic function, particularly in the regions close to the stent pillars at the commissures. These regions, also referred to as regions of blood stagnation, may encourage local thrombogenesis, tears and calcifications, and may lead to structural deterioration in the longer term.

This transcatheter procedure, since the early year 2000, is growing fast and gradually replacing the surgical procedures but unfortunately the TAVI are not free from clinical complications that in part are due to the presence of the native stenotic valve.

On the contrary, the surgical replacement is free from this inconvenience since the native diseased valve is manually removed but as discussed before, the procedure is highly invasive.

Since the first-in-human procedure in 2002, TAVI has become a well-established therapeutic option for severe aortic stenosis and is offered increasingly to patients at lower surgical risk, who are typically younger. Increasing lifespan carries concerns that "minor" complications that may have little impact on elderly patients could have a greater long-term impact on younger patients. Issues such as mild paravalvular regurgitation, hypo attenuated leaflet thickening, atrioventricular block with need for permanent pacemaker implantation or difficult access to treat coronary arteries may have a substantial cumulative undesirable impact. Additionally, as happens with surgical bioprosthetic valves, transcatheter bioprosthetic valves will eventually degenerate, and may require repeating the intervention. Although haemodynamic data for transcatheter heart valves (THVs) seem encouraging, long-term data on clinical durability are controversial. Redo TAVI (so called TAVI in TAVI) has been shown to be feasible with acceptable outcomes in patients with failed TAVI, but in some patients, anatomical or device considerations may preclude a repeatable procedure because of the risk of coronary arteries obstruction and patient's mismatch sizing. Various strategies for lifetime management in this lower-risk and younger patient population have been proposed: surgical aortic valve replacement (SAVR) first, followed by TAVI; TAVI then SAVR; TAVI then TAVI, etc. A tailored approach may be considered according to patient co-morbidities, anatomy and the relative advantages and disadvantages of the two therapies and life expectancy. To answer in a safe and effective way this need for an early heart valve replacement in patient populations younger and at lower operative risk, a brand-new heart bioprosthetic valve should be developed.

Under such circumstances, a good compromise should be found. Create a procedure with minimal invasiveness joined to the possibility of removing the native diseased heart valve through a minimally invasive procedure such as a transcatheter intervention. This condition should be ideal for reducing risks related to a surgical operation and increasing life expectancy even in young patients with stenotic diseases.

There is, therefore, a need to improve existing transcatheter procedures for anchoring heart valve prostheses.

### Summary of the Invention

The present invention provides an improvement with respect to existing transcatheter procedures.

To this effect, the invention may advantageously be used in a transcatheter procedure where the native valve leaflets have been previously fully excised.

More precisely, the invention consists of a sutureless transcatheter heart valve prosthesis comprising a collapsible stent structure with anchoring elements and a valve component located within said stent structure. The heart valve prosthesis according to the invention is characterized by the fact that the stent structure is configured to be positioned within a heart valve annulus after full excision of the native valve leaflets; the said stent structure comprising a sub-annular anchoring element, a supra-annular anchoring element, and wherein said sub- and supra-annular anchoring elements are configured to cinch ridges that are formed by the valve commissures and the resection profile after the full excision of said native valve leaflets.

The heart valve prosthesis according to the invention, may be efficiently anchored to the quasi-circular annulus that is obtained after full excision of the native valve leaflets. This quasi-circular annulus forms a circular rim that provides an optimal implant site for heart valve prosthesis according to the invention. The heart valve prosthesis according to the invention, when compared to existing TAVI heart valve prostheses, provides a larger orifice area and a reduced number of drawback clinical complications observed after TAVI procedures.

Any suitable transcatheter device can be used for the resection of valve calcified leaflets.

To enable a safe and effective prosthetic valve deployment, after excision and removal of the calcified valve leaflets, the transcatheter excision device, preferably also provides a temporary valve function.

The transcatheter heart valve prosthesis may be advantageously deployed when the resection device, with a temporary valve function procedure is ongoing. The temporary valve function of the excision device should maintain a stable blood perfusion during the positioning and deployment of the present invention.

According to a preferred embodiment, the heart valve prosthesis according to the invention is an aortic valve prosthesis. In this case the stent structure is configured to be positioned within an aortic root and the said stent structure furthermore comprises a sinusal anchoring element.

The heart valve prosthesis according to the invention may be used for replacing any type of heart valve, particularly an atrioventricular valve, especially a mitral valve.

This heart valve prosthesis according to the invention solves or mitigates at least the following important TAVI related clinical drawbacks:
- Paravalvular regurgitation/leakages due to a sub-optimal (irregular or incomplete) coupling between the deployed TAVI and the stenotic leaflets leaving empty spaces leakaging blood in systole and in diastole;
- Premature structural deterioration related to the non-circular deployment of the TAVI, inside the stenotic native valve, with altered functional opening/closure of the prosthetic leaflets;
- Thrombotic clots, collagen and calcium embolization from the stenotic native valve during and after few years after TAVI deployment procedure;
- Reduction of the EOA (Effective Orifice Area) with the condition of patient-prosthesis mismatch occurring when a TAVI is placed in degenerated surgical bioprostheses or TAVIs;
- Not negligible rate (20%) of permanent pacemaker implantation due to atrio-ventricular conduction damages during TAVI procedures despite the improvements in procedural success;
- Difficult access to coronary arteries, for angioplasty procedures, after TAVI due to the outward encumbrance of native leaflets and narrowing of Valsalva Sinuses.

The heart valve prosthesis according to the invention is conceived with a dedicated anatomical anchoring system, designed to anchor the valve at native leaflets' resected edges, valve annulus and, if it is an aortic valve prosthesis, coronary sinuses and sinotubular junction. An anatomical anchoring system means a specific stent structure designed to perfectly match with the native valve annulus size and possibly ensure accurate alignment with native valve commissures, fitting and sealing to the aortic root during the deployment of the prosthesis. Advantageously the prosthetic valve has different sizes in diameter to allow the best fitting to different patients' native annuli.

In one specific embodiment of the present invention, the prosthetic valve used in the aortic position has a particular stent design that fits the anatomical features of the native aortic valve (e.g., annulus size and diameter, coronary sinuses conformation, coronary ostia height from the annulus etc.).

The absence of the stenotic native leaflets is demanding to design specific anchoring structures at the annulus level such as paddles and/or engagement arms and/or skirts opened outward from the stent structure that are used to anchor the prosthesis, to prevent paravalvular leakages in the long-term and to ensure a perfect sealing at valve inflow. To anchor the valve at the valve annulus level, the stent could have "V" shape structures bent outward the frame of the stent or eventually "rhombus" shape ring structures with engagement arms bending outward the stent.

In one embodiment, the bended stent structures at the level of valve annulus are coated to reduce possible anatomical damages granting prevention from paravalvular leakages and ensuring perfect sealing. In another embodiment of the present invention all bended outward structures of the valve stent, (paddles, engagement arms, skirt etc.), used to enhance anchoring with the anatomy, could be coated. The coating used to cover bended stent structures could be made by biological tissues or polymeric fabrics or polymeric bulk materials/films.

Not only bended stent structures of the valve are coated. In one embodiment, specific areas of the stent valve are coated to enhance anatomical grip and sealing.

In one embodiment, the flared ventricular stent structures at inflow side of the present invention are asymmetrical to allow perfect anatomical conformation and preventing possible interference with the mitral valve creating flow decompensation.

The valve could be self-expandable and realized in NiTinol or eventually balloon-expandable and realized by Chromium-Cobalt alloy or similar. In one embodiment, the balloon used to open the valve or specific part of the valve (e.g. bended stent structures) could be toroidal to expand the stent valve structure in a pre-defined shape. In another embodiment, the valve could be expanded via another mechanical system.

In another embodiment, the balloon-expandable prosthetic valve could be deployed with two or three balloons inflated subsequently during the procedure (first the deployment at annulus level and second the deployment at sinotubular junction level to stabilize the prosthesis). These balloons can be mounted on the same delivery system's shaft.

Some of self-expandable TAVI systems, currently in clinical use, are causing ischemic compression of A-V node at mitro-aortic continuity. The mitro-aortic continuity compression is responsible for atrio-ventricular node conduction disturbances often requiring a pacemaker implantation. Therefore, the choice of a balloon-expandable design could be supported by the current TAVI studies in which, for this type of TAVI, electrical A-V conduction problems have a lower rate of occurrence. These A-V conduction issues are possibly related to the dynamic continuing compression over the calcific tissue of the native aortic valve exerted by self-expandable prosthetic valves with the stent frames made by NiTinol alloy.

In one embodiment of the present invention the balloon-expandable prosthetic valve could be realized with a low profile. This is the option where the stent is exclusively anchored at annulus level and at leaflets commissural profiles, leaving more space in case of future coronaries accesses for angioplasty procedures.

In another embodiment, the balloon-expandable prosthetic valve could be realized with high profile. This option is used in case the valve is requiring additional anchoring at sinotubular junction to avoid tilting of the prosthesis.

In synthesis the sutureless transcatheter aortic valve prosthesis, independently is balloon- or self-expandable is designed with three levels of flanges or anchoring systems (from inflow to outflow): one on the ventricular side, the sub-annular anchor, a second one on the aortic side, the supra-annular anchor and a third one at sinotubular junction, the sinusal anchor. The first two are granting a cinching and sealing at level of the aortic root rim left after excision of the native stenotic valve. The third one is a flare of the outflow prosthetic pillars that can be shaped and extended with different solutions of engagement and stabilizer arms at level of sinotubular junction. The first two levels of anchoring are providing a sealing and stabilization of the prosthesis preventing migration and paravalvular leakages while the third level is preventing the tilting movements. The anchoring system, in case of self-expandable prosthesis, should also allow the prosthesis to be recaptured for a possible repositioning.

The invention preferably comprises an assembly representing the valve function that can be realized with one or more leaflets, preferably tri-leaflet, and is mounted inside the stent frame. The material used for the functional assembly can be made of biologic tissues such as swine or bovine pericardium or by polymeric films. A specific crosslink or post-crosslink treatment on biological tissue could be applied or polymeric material could be selected to grant the best biocompatibility, resistance to fatigue and to prevent long-term thrombotic risk and stenosis due to calcium formation.

The invention also preferably concerns to a coated heart valve as it is for TAVIs. The coating is made by biologic tissues such as swine or bovine pericardium or by polymeric films or fabrics used in a fashion of a skirt to guarantee the sealing between the stent structure and the aortic root anatomy. The stent coating is required for two main functions: completely seal the annulus preventing paravalvular leakages and gain additional grip to maintain the valve stable and in the preferable deployed position.

Depending on valve design, not all stent valve structures are coated. Coating is mainly adopted where it's necessary to seal and increase grip, but it's not foreseen in areas where are located for example coronary arteries. The valve should always guarantee a perfect coronary perfusion and possible future accesses for transcatheter coronary interventions.

Radio-opaque markers on stent valve structure are also used to facilitate deployment of the anchoring system and the rotational orientation whenever it is needed under Fluoroscopy or Echo imaging. The present sutureless transcatheter heart valve prosthesis, after its own transcatheter implant, is also ensuring an optimal hemodynamic flow thanks to the tri-leaflets valve apparatus.

The sutureless transcatheter heart valve prosthesis's delivery system is realized adopting technical features specifically adapted to this valve prosthesis. The sutureless transcatheter heart valve prosthesis of the present invention is deployed with the help of a dedicated steerability of the outer catheter (e.g. four degrees of freedom with multiple handles control) able to drive the prosthesis in-situ, allowing axial rotation and longitudinal movements for the best positioning. In one embodiment, the delivery system axial rotation feature is necessary to perfectly align the valve prosthesis structure with the native valve commissural profiles.

Before prosthetic valve implantation, as for TAVI procedures, the valve prosthesis of the present invention is crimped down and loaded into a dedicated stent cover at the distal part of the delivery system. In one embodiment, with a self-expandable prosthetic valve, the stent cover can be single or in two parts to guarantee a more precise valve deployment. With two parts stent cover the sliding of distal part of stent cover is deploying the ventricular (inflow) side of the valve, sliding of proximal part of the stent cover is deploying the aortic (outflow) side of the valve prosthesis. The double stent cover option is also ensuring re-capturability of the valve when mispositioning may occur. In addition, the stent cover can be provided with radio-opaque markers to facilitate the procedure of valve deployment and alignment to the aortic root anatomy.

In another embodiment with a balloon-expandable valve, the prosthetic valve is crimped down over a shaft carrying on one or more balloons placed sequentially. The deployment of the prosthetic valve, when the delivery system is in position, is occurring with on single inflation or with two sequential inflations (the first one to deploy the annulus portion (sub- and supra-annular) and the second one to deploy the sinotubular portion. The double balloon expansion can be obtained with two single sequential balloons, independently controlled or with a single balloon having an internal partition in a way to act as a double balloon.

### Detailed description of the invention

The invention will be better understood in the present chapter, with some non-limiting illustrated examples that refer to an aortic valve prosthesis.

### Brief description of the figures

Figure 1a native aortic valve with calcified leaflets.
Figure 1b native aortic valve after calcified native leaflet resection.
Figure 2 example of aortic root with the aortic leaflets.
Figure 3 TAVI deployed inside a calcific aortic valve.
Figure 4 example of aortic root in which the native aortic valve leaflets have been excised.
Figure 5 Sutureless prosthetic valve with its three levels of anchoring deployed inside an aortic annulus without the native leaflets.
Figure 6 Flat design of a self-expandable stent with asymmetric sub-annular inflow structures.
Figure 7 3D configuration of a self-expandable stent represented in

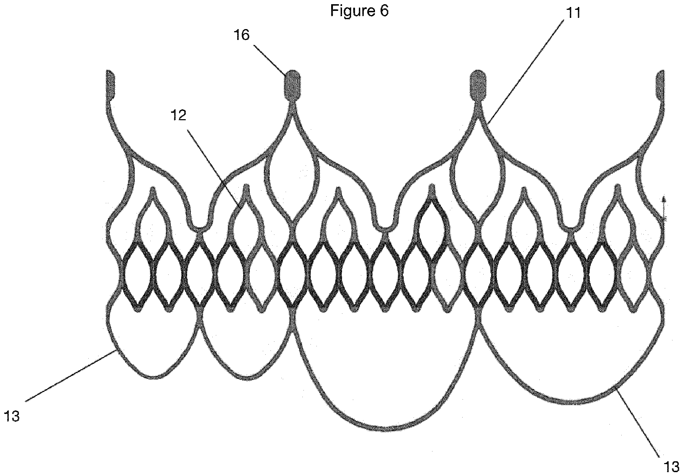

Figure 6. Visible the flared supra-annular structures and asymmetric sub-annular structures.

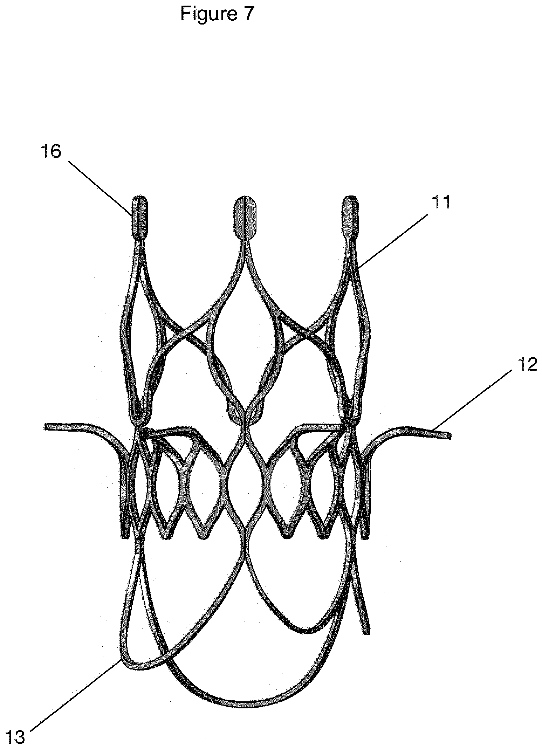

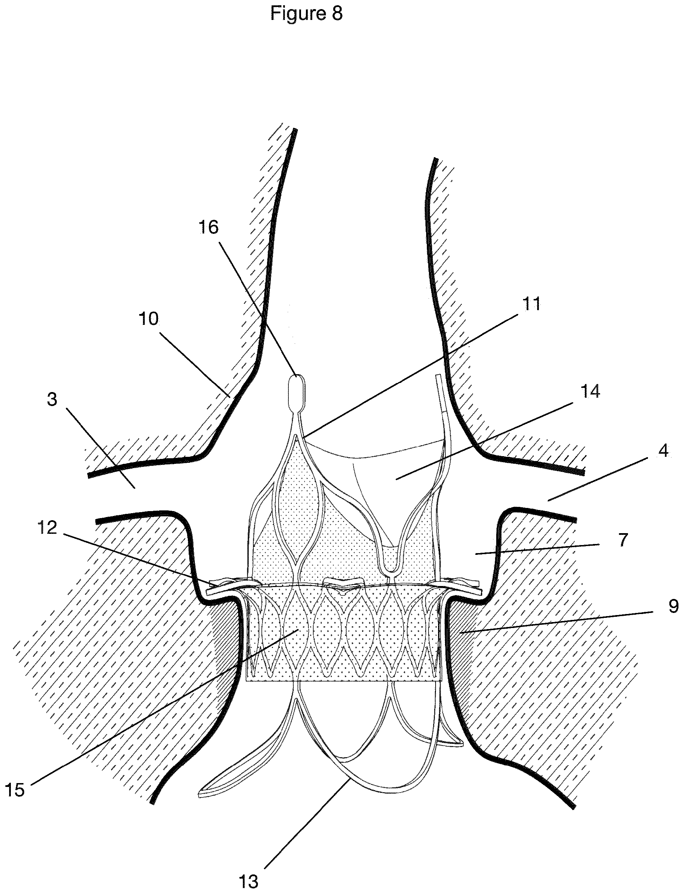

Figure 8 Self-expandable prosthetic valve as described in Figure 6 and 7 deployed in the aortic root. Pericardial tissue inside and cloth made of biologic or polymeric material.

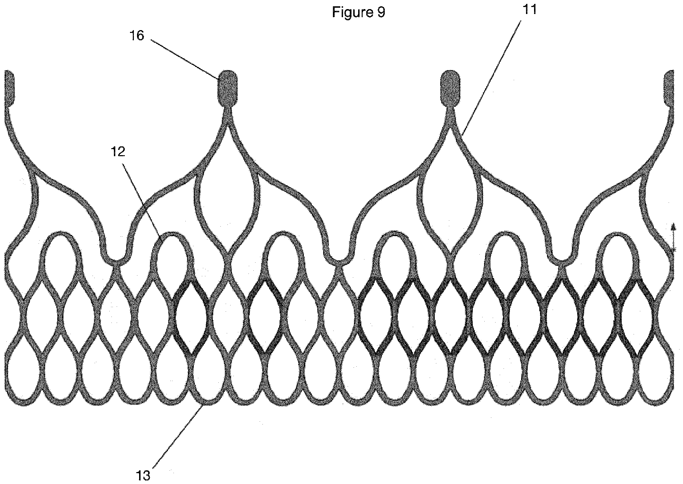

Figure 9 In this embodiment a self-expandable stent in flat design is represented.

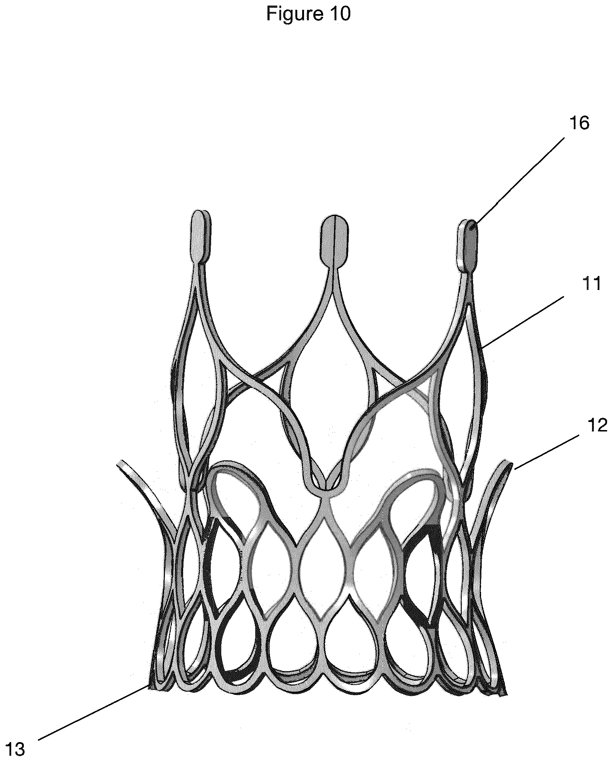

Figure 10 a self-expandable stent structure as per Figure 9. The sub- and supra-annular anchoring is granted by a flaring of the stent structures. The sinotubular anchoring is granted by upper structures bent outwards and placed on the outflow portion of the stent.

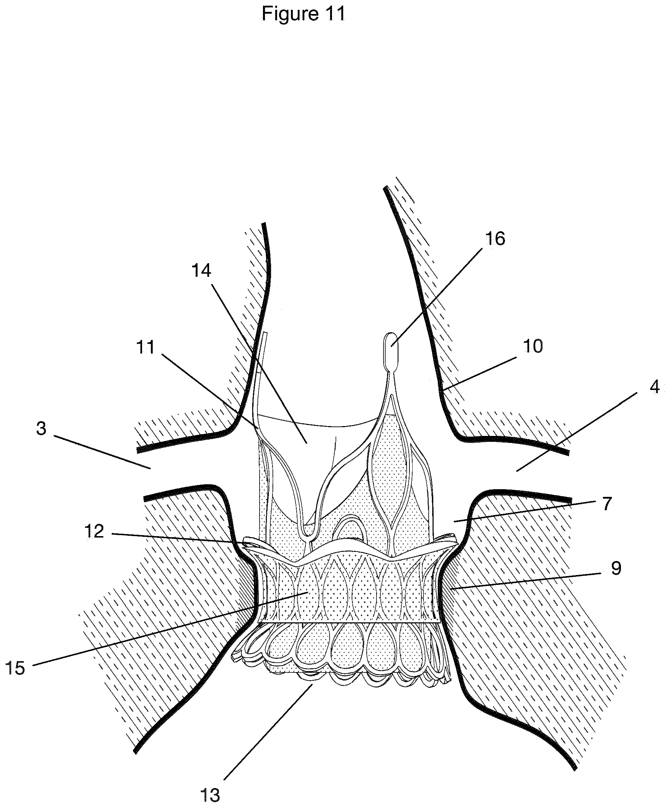

Figure 11 The assembled prosthetic valve as represented in Figures 9 and 10 deployed in the aortic root. Pericardial tissue inside and cloth made of biologic or polymeric material. This prosthetic valve has an asymmetric sub-annular anchoring flare (variable angle of flare or length of the structures).

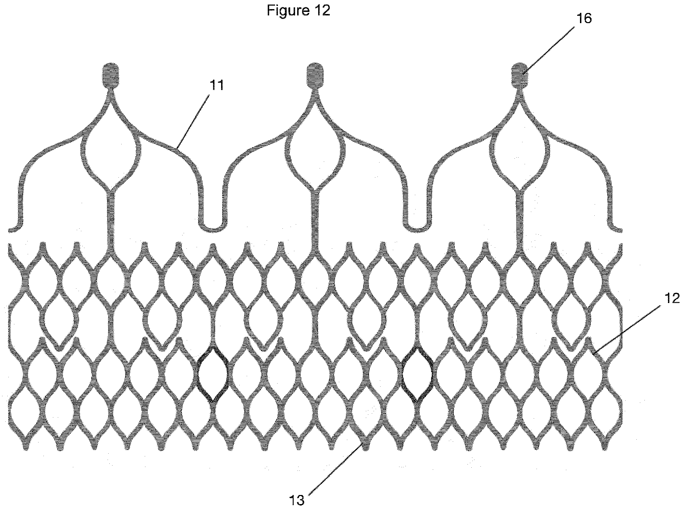

Figure 12 Flat design of a self-expandable stent. The bottom portion (inflow) can be flared out (sub-annular anchor), the middle portion has structures that can be bent outwards (supra-annular anchor). The sinusal anchoring is obtained with the upper structures with elements that can be bent outwards to get in contact with the aorta's wall.

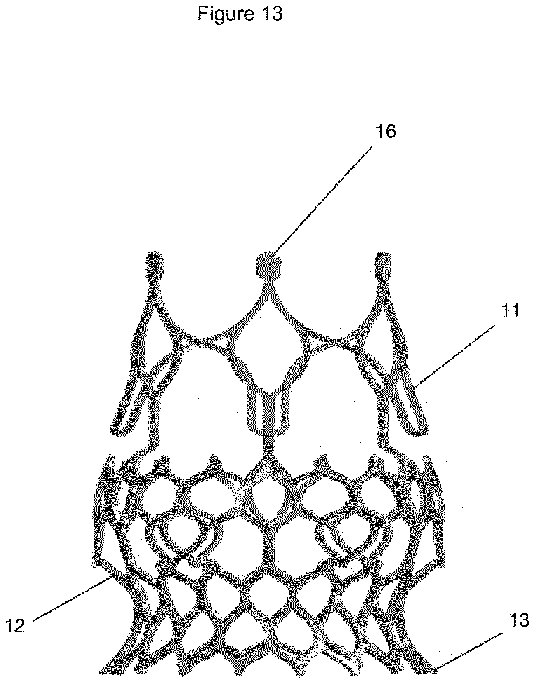

Figure 13 3D configuration of the self-expandable stent represented in Figure 12. Visible the flared sub-annular structures and the bent supra-annular structures. The sinusal anchoring is granted by the bent out "V" shaped structures.

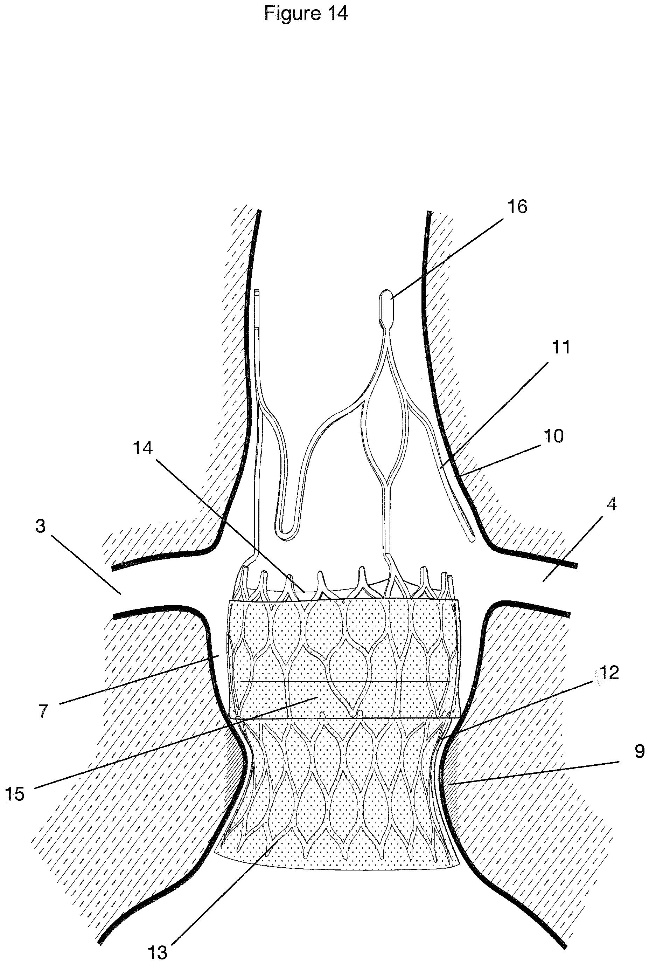

Figure 14 the assembled prosthetic valve as represented in Figures 12 and 13 deployed in the aortic root. Pericardial tissue outside and cloth made of biologic or polymeric material. This prosthetic valve has a symmetric sub-annular anchoring flare.

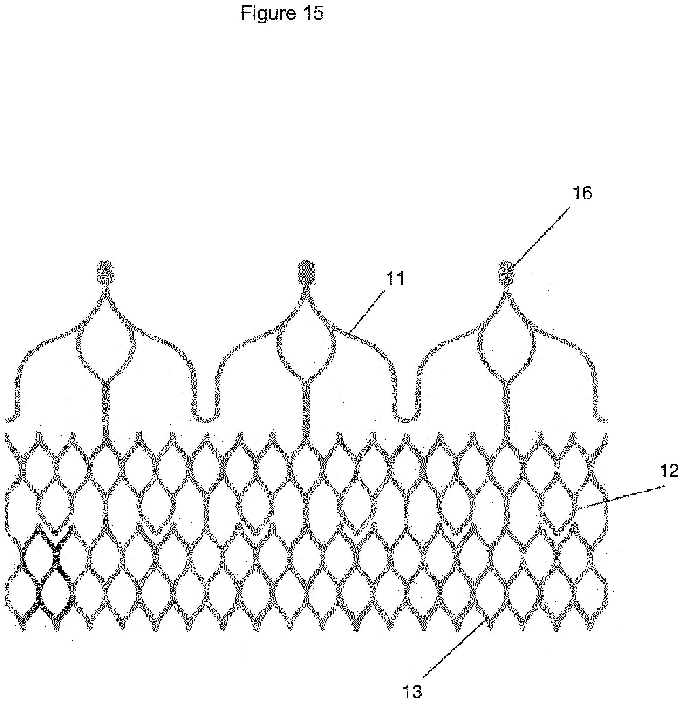

Figure 15 Flat design of a self-expandable stent. The bottom portion (inflow) can be flared out (sub-annular anchor), the middle portion has structures that can be bent outwards (supra-annular anchor). The sinusal anchoring is obtained with the upper structures with elements that can be bent outwards to get in contact with the aorta's wall.

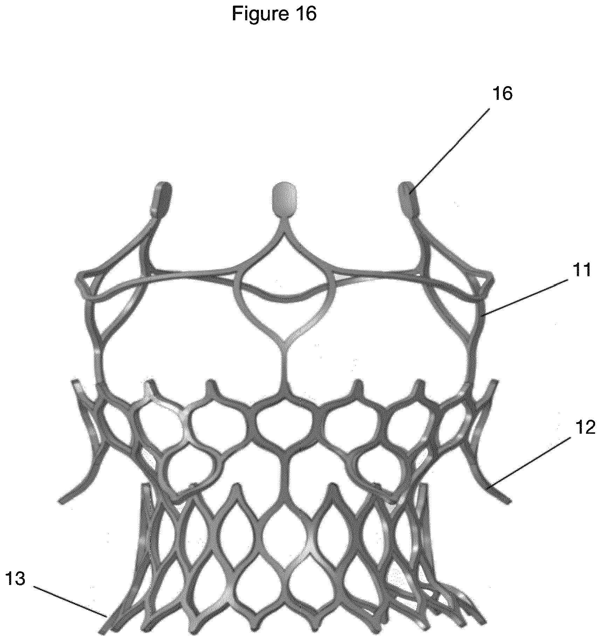

Figure 16 3D configuration of the self-expandable stent represented in Figure 15. Visible the flared sub-annular structures and the bent supra-annular structures. The sinusal anchoring is granted by the bent out "V" shaped structures.

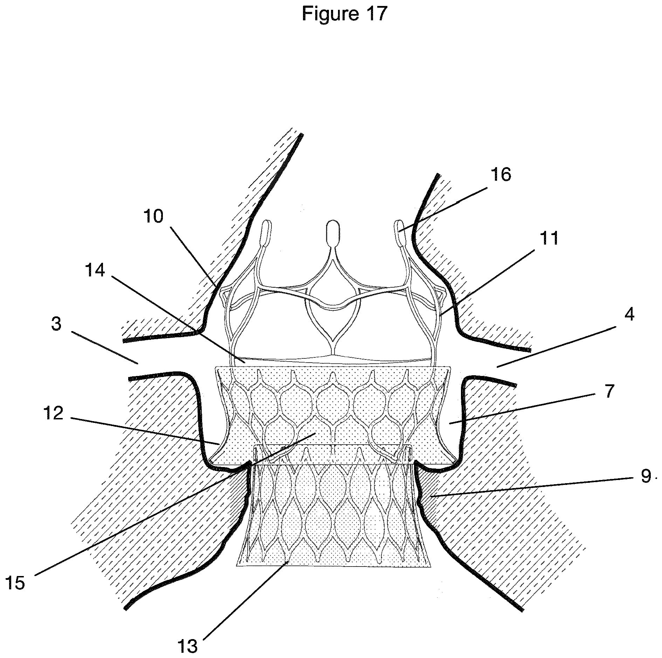

Figure 17 the assembled prosthetic valve as represented in Figures 16 deployed in the aortic root. Pericardial tissue outside and cloth made of biologic or polymeric material. This prosthetic valve has a symmetric sub-annular and supra-annular anchoring flare. The sinusal anchoring part of the stent (outflow) is not covered by tissue and helps valve stability thanks to "V" shaped structures bent out towards the aorta.

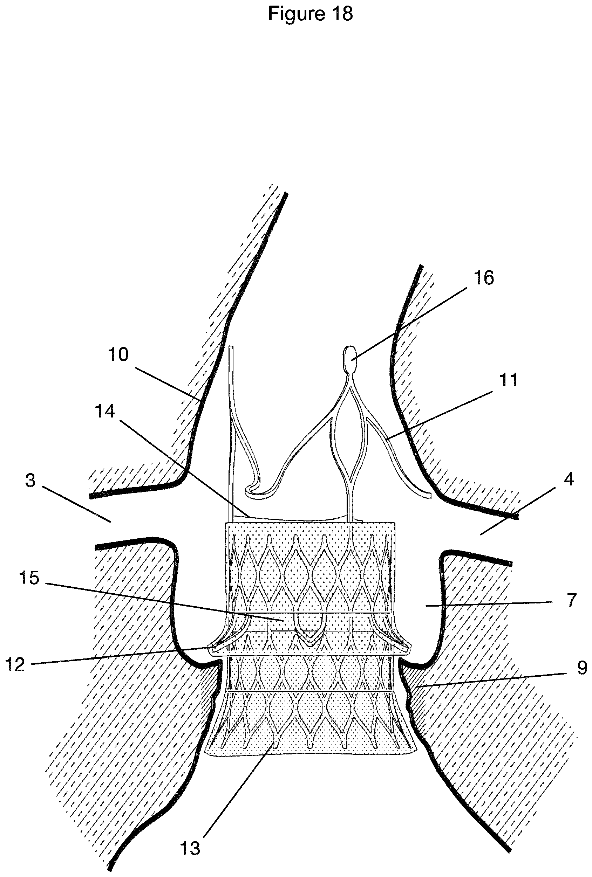

Figure 18 an alternative deployment of prosthetic valve, represented in Figures 16, in the aortic root. Pericardial tissue outside and cloth made of biologic or polymeric material. This prosthetic valve has a symmetric sub-annular and supra-annular anchoring flare. The supra-annular anchors are less opened and covered by a tissue skirt used to prevent paravalvular leaks. The sinusal anchoring part of the stent (outflow) is not covered by tissue and helps valve stability thanks to "V" shaped structures bent out towards the aorta.

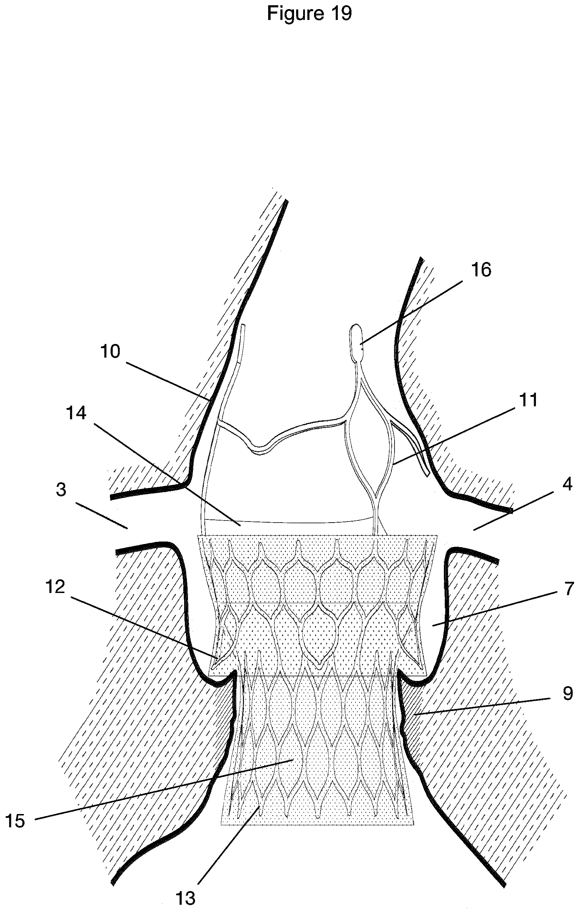

Figure 19 an additional deployment of prosthetic valve, represented in Figures 16, in the aortic root. As per Figure 18, the sub-annular and supra-annular covered stent structures are less bended out.

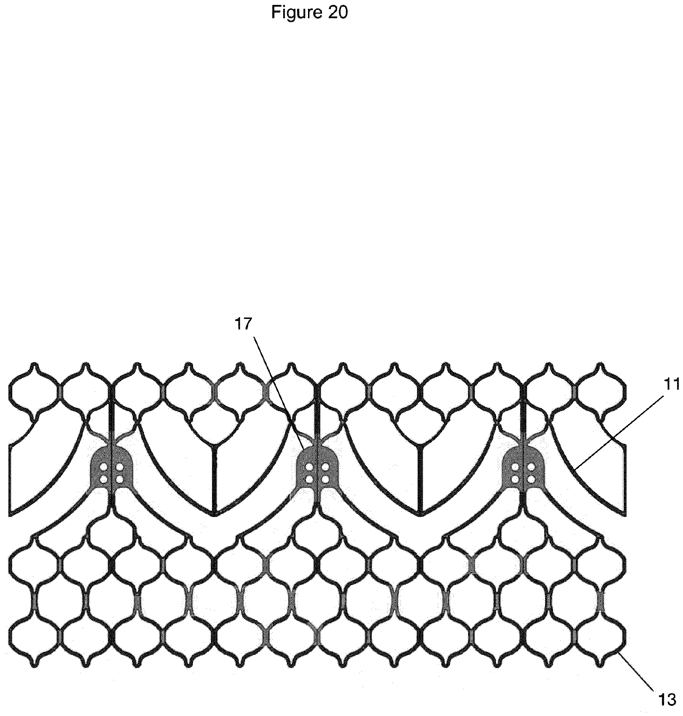

Figure 20 Flat design of a balloon-expandable stent. The bottom portion (inflow) can be gently flared out (sub-annular anchor), the middle portion has no bended out structures. The sinusal anchoring is obtained with the upper structures with elements that can be bent outwards to get in contact with the aorta's wall to increase stability.

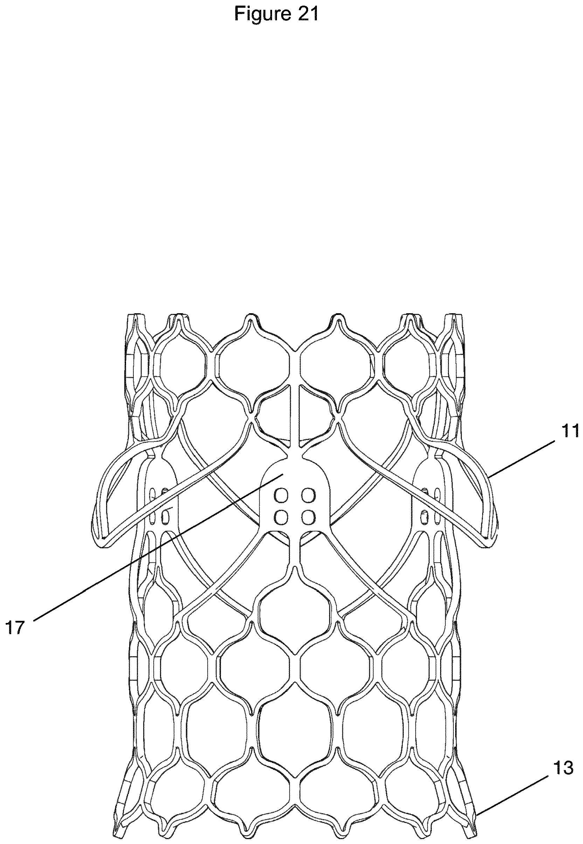

Figure 21 3D configuration of the balloon-expandable stent represented in Figure 20. Visible the flared sub-annular structures and the bent sinusal structures. The sinusal anchoring is granted by the bent out "V" shaped structures.

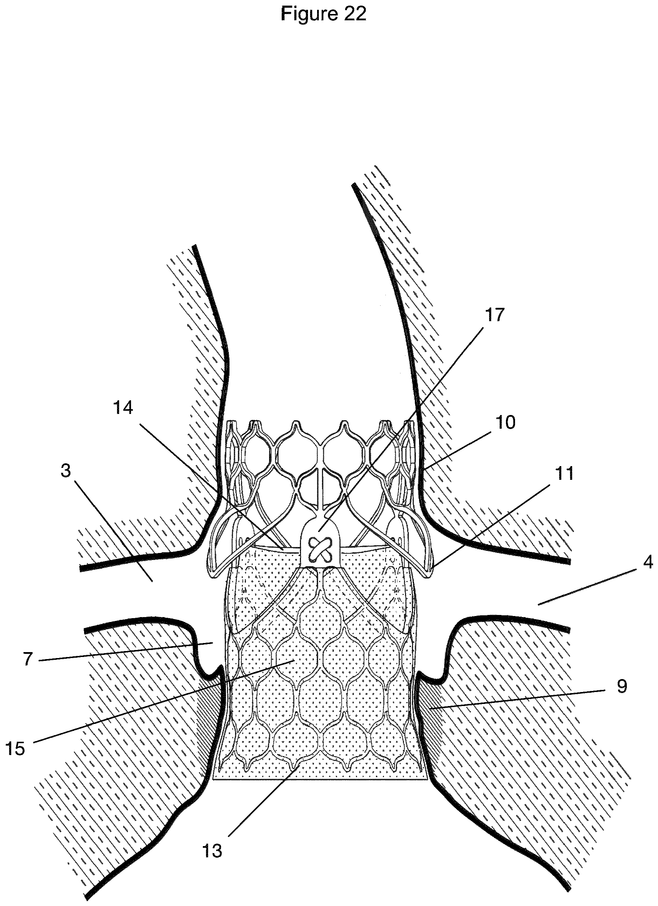

Figure 22 the assembled prosthetic valve as represented in Figures 21 deployed in the aortic root. Pericardial tissue outside and cloth made of biologic or polymeric material. This prosthetic valve has a symmetric sub-annular and sinusal anchoring flare. The sinusal anchoring part of the stent (outflow) is not covered by tissue and helps valve stability thanks to "V" shaped structures bent out towards the aorta.

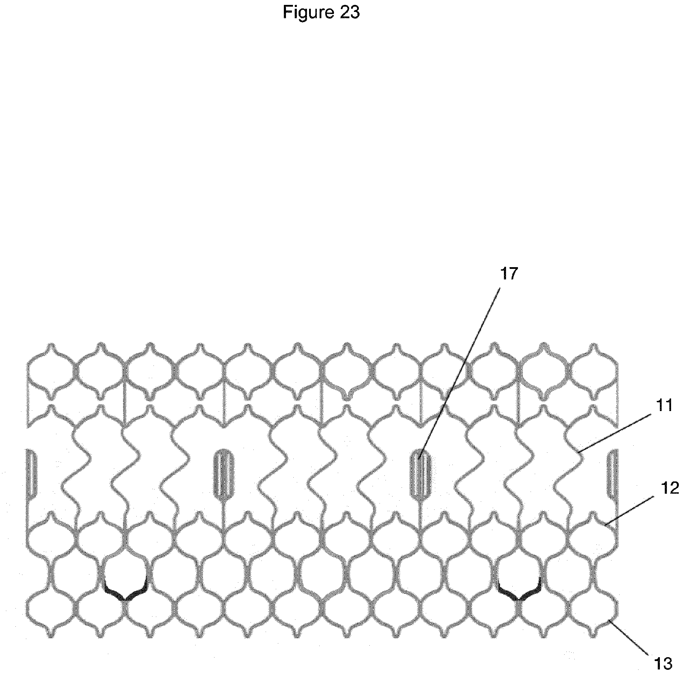

Figure 23 Flat design of a balloon-expandable stent. The bottom portion (inflow) can be flared out (sub-annular anchor), the middle portion can be flared out too (supra-annular anchor). In addition, the sinusal anchoring is obtained with the upper structures with elements that can be bent outwards to get in contact with the aortic roots to increase stability.

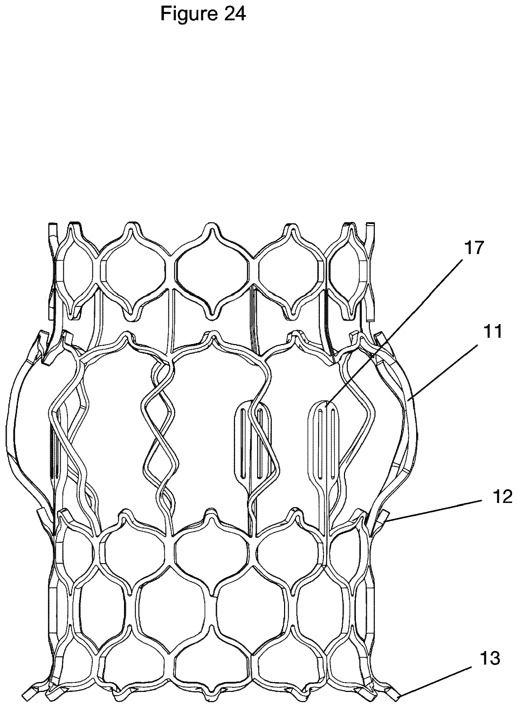

Figure 24 3D configuration of the balloon-expandable stent represented in Figure 23. Visible the flared sub-annular and supra-annular structures to get in contact with the aortic annulus (cinching grip (C shape) at annulus level). The sinusal anchoring is granted by the bent out shaped structures in contact with aortic roots.

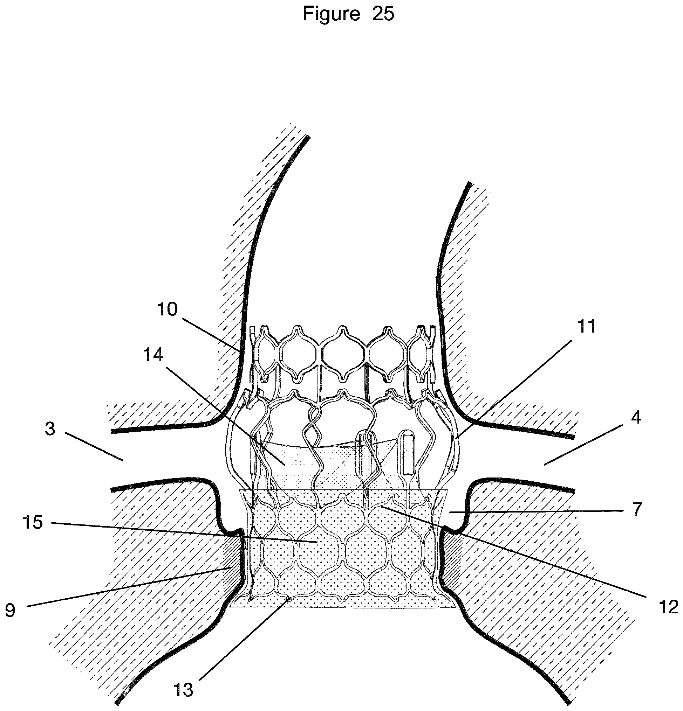

Figure 25 the assembled prosthetic valve as represented in Figures 24 deployed in the aortic root. Pericardial tissue outside and cloth made of biologic or polymeric material. This prosthetic valve has a symmetric sub-annular, supra-annular and sinusal anchoring flare. The sinusal anchoring part of the stent (outflow) is not covered by tissue to prevent coronaries obstructions and helps valve stability.

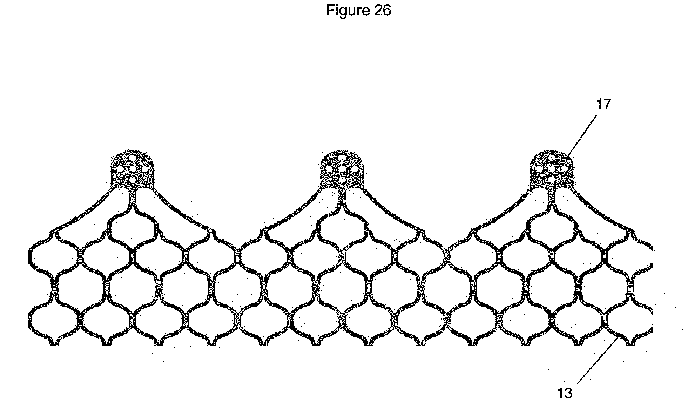

Figure 26 Flat design of a low-profile balloon-expandable stent. The bottom portion (inflow) can be flared out (sub-annular anchor). No additional middle portion (supra-annular anchor) and sinusal elements used to anchor the valve.

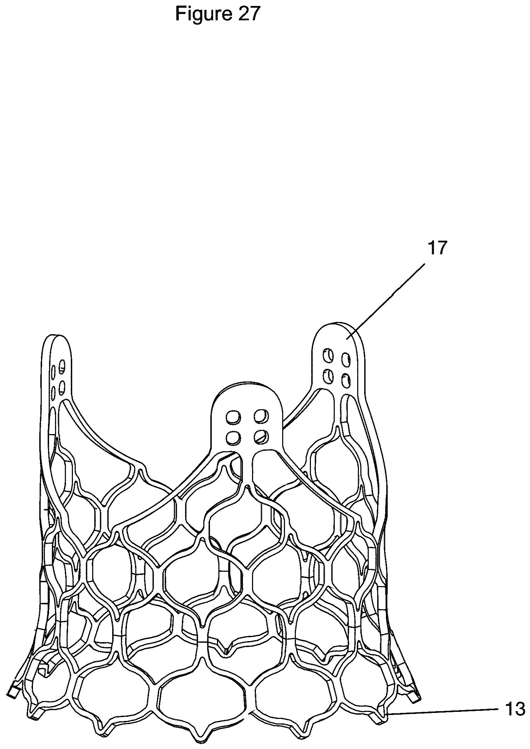

Figure 27 3D configuration of the balloon-expandable stent represented in Figure 26. Visible the unique flared sub-annular structures to get in contact with the aortic annulus. No supra-annular and sinusal elements are used to anchor the valve in the aortic root.

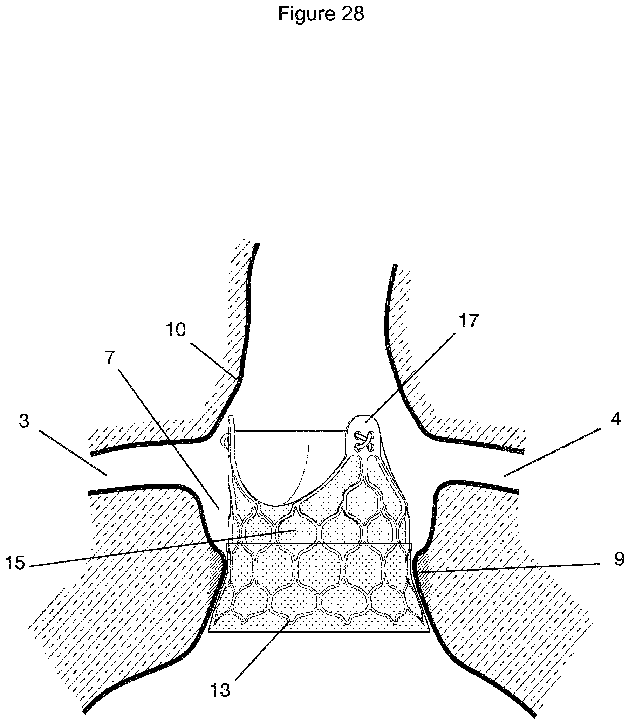

Figure 28 the assembled prosthetic valve as represented in Figures 27 deployed in the aortic root. Pericardial tissue outside and cloth made of biologic or polymeric material. This prosthetic valve has a symmetric sub-annular anchoring flare. The low-profile stent structure is all covered by pericardial tissue to prevent paravalvular leakages.

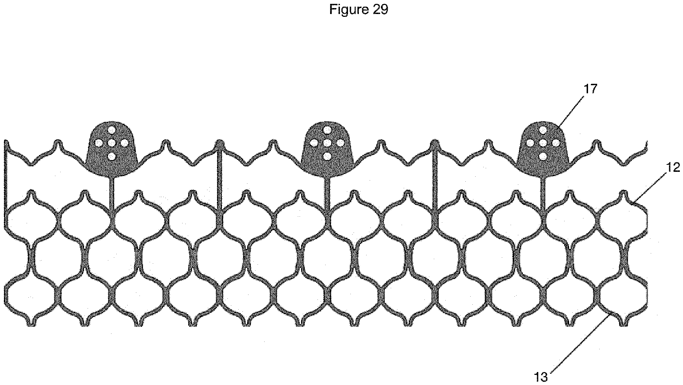

Figure 29 Flat design of a low-profile balloon-expandable stent. The bottom portion (inflow) and upper portion can be flared out (sub and supra-annular anchor) to anchor the stent in the aortic annulus. No additional sinusal elements used to anchor the valve.

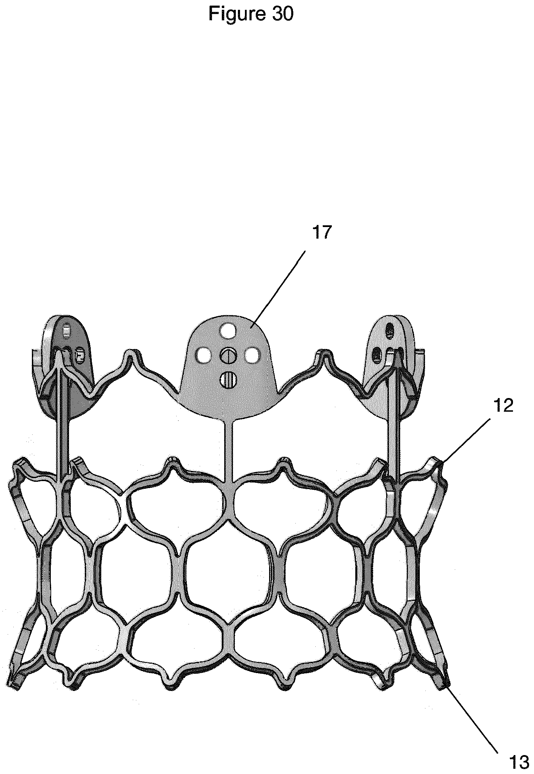

Figure 30 3D configuration of the balloon-expandable stent represented in Figure 29. Visible the flared sub-annular and supra-annular structures to get in contact with the aortic annulus (cinching ridges that are formed after the full excision of said native valve leaflets) . No sinusal elements are used to anchor the valve in the aortic root.

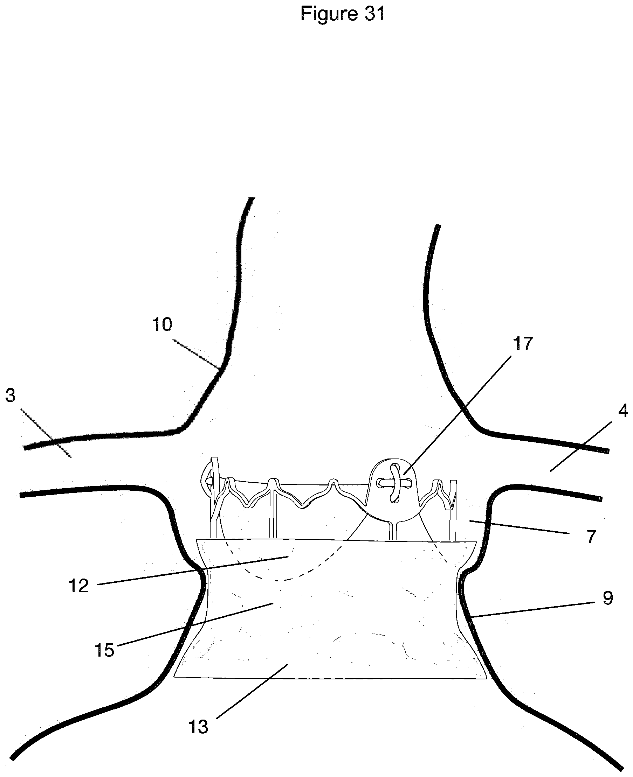

Figure 31 the assembled prosthetic valve as represented in Figures 30 deployed in the aortic root. Pericardial tissue outside and cloth made of biologic or polymeric material. This prosthetic valve has a symmetric sub-annular and supra-annular anchoring flare. The low-profile stent structure is all covered by pericardial tissue or polymeric material to prevent paravalvular leakages.

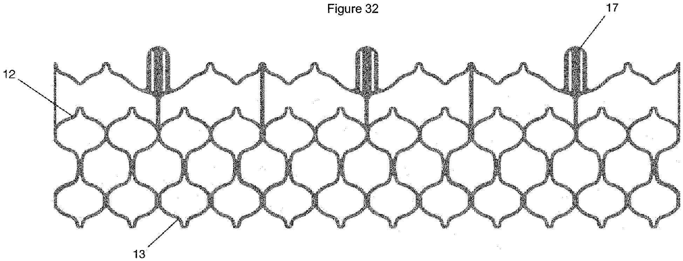

Figure 32 Flat design of a low-profile balloon-expandable stent. The bottom portion (inflow) and supra-annular portion can be flared out (sub and supra-annular anchor) to anchor the stent in the aortic annulus. No additional sinusal elements used to anchor the valve.

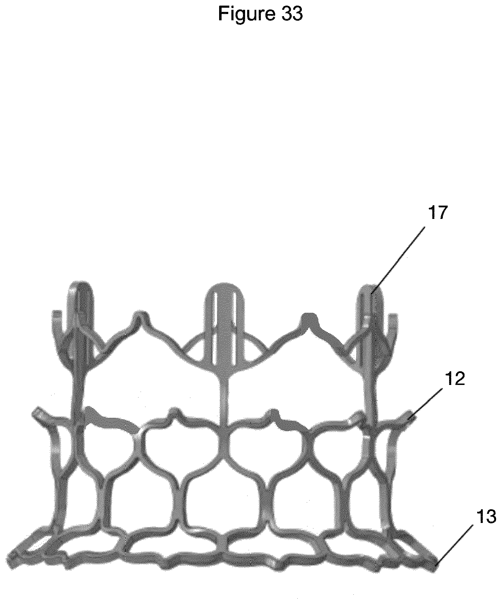

Figure 33 3D configuration of the balloon-expandable stent represented in Figure 32. Visible the flared sub-annular and supra-annular structures to get in contact with the aortic annulus (cinching ridges that are formed after the full excision of said native valve leaflets) . No sinusal elements are used to anchor the valve in the aortic root.

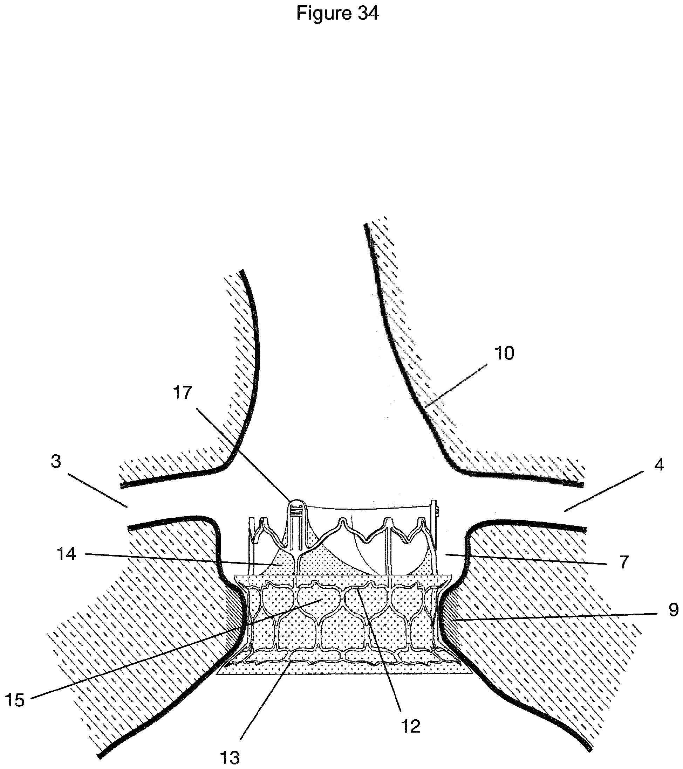

Figure 34 the assembled prosthetic valve as represented in Figures 33 deployed in the aortic root. Pericardial tissue outside and cloth made of biologic or polymeric material. This prosthetic valve has a symmetric sub-annular and supra-annular anchoring flare. The low-profile stent structure is all covered by pericardial tissue or polymeric material to prevent paravalvular leakages.

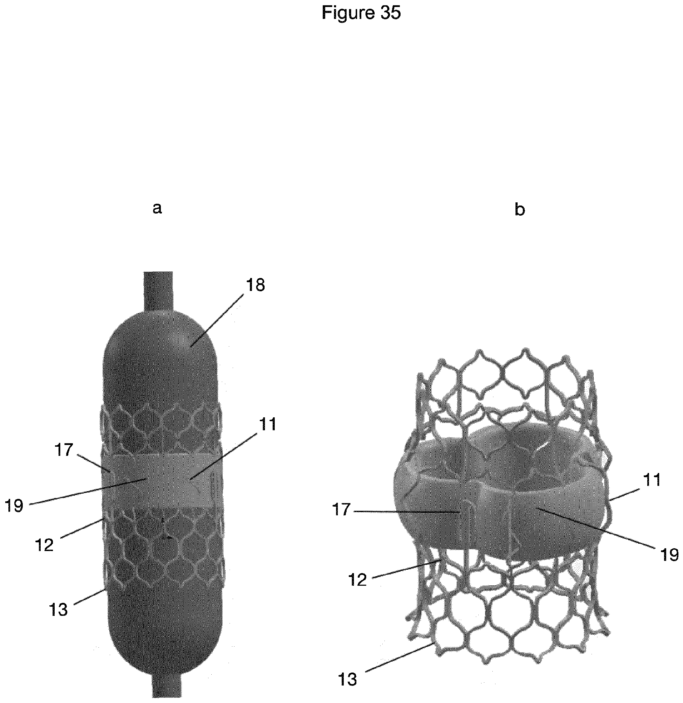

Figure 35 pre and post configuration of the balloon-expandable stent represented in Figure 24. The trilobed shaped balloon is granting a perfect expansion of the stent at the aortic roots level.

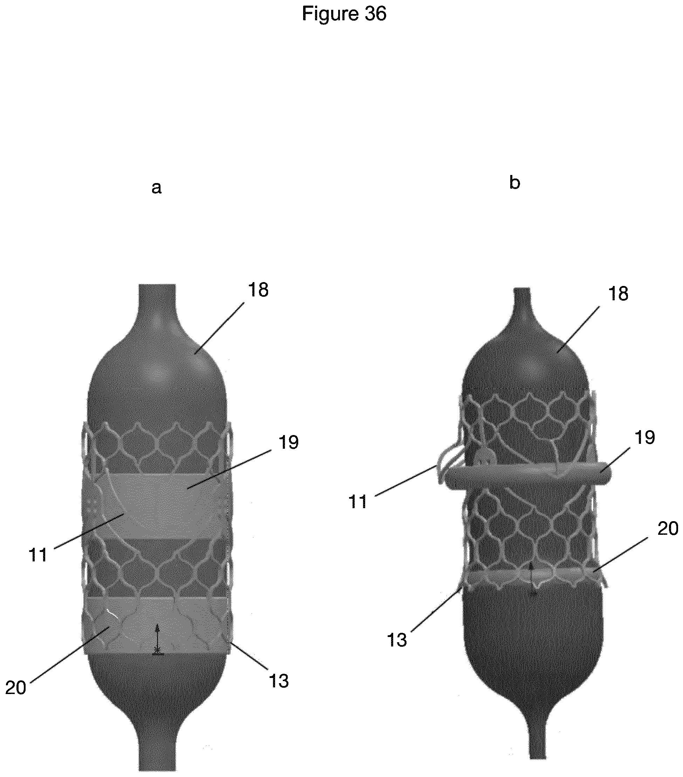

Figure 36 pre and post configuration of the balloon-expandable stent represented in Figure 21. The doubled shaped balloon is granting a perfect expansion of the stent at sub-annular and sinusal level.

### Numerical references used in the figures

1.Aortic valve leaflet
2.Aortic valve commissure
3.Right coronary artery
4.Left coronary artery
5.Aortic annulus resection profile
6.Aortic valve outflow
7.Coronary sinuses
8.Aortic valve inflow
9.Aortic annulus (anatomical part composed by resected native valve commissures and the resection profile of the native valve leaflets)
10.Sinotubular junction (anatomy that links the Valsalva sinus with the ascending aorta)
11.Sinusal anchoring structures
12.Supra-annular anchoring flare
13.Sub-annular anchoring flare
14.Pericardial or polymeric tissue leaflets
15. External pericardial or polymeric tissue cover
16.Radiopaque markers
17.Stent structure to sustain leaflet commissure stitches
18.Overall stent expansion balloon
19.Sinusal stent level expansion balloon
20.Sub-annular stent level expansion balloon

### Detailed description of the figures

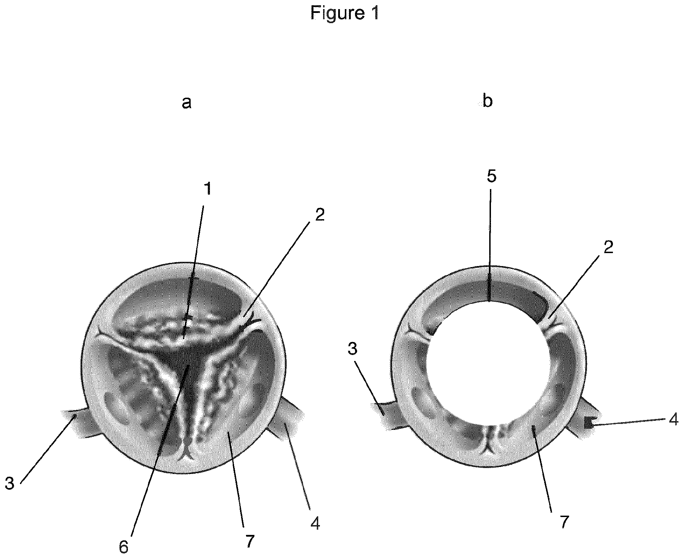

In Figure 1a is possible to observe the stenotic pattern of an aortic heart valve in which the calcification of the leaflet 1 is spread along the three valve leaflets. The calcification 1 process starts in the low middle portion of each belly looking from the aortic valve outflow 6. Calcifications of the leaflets 1 are irregular, and gradually extend to valve commissures 2 in one side and up to the top of each belly in the other side. They assume, in each leaflet the aspect of a semilunar shape with larger part in the belly of each leaflet.

Dystrophic fibrosis and calcifications of leaflets 1 are responsible of tissue stiffening with narrowing of the valve orifice 6 and high systolic pressures. In diastole the leaflets, due to the stiffening, are unable to perfectly coapt and a variable grade of regurgitation into the left ventricle is occurring.

The aortic prosthetic valve, presented in this patent, is conceived to be implanted in a resected aortic annulus orifice 5 where the leaflets 1 are not anymore present (Figure 1b). The excision can be conducted with a dedicated transcatheter system without damaging the aortic wall, the Valsalva coronary sinuses 7, the commissures 2 and occluding the right 3 or the left 4 coronary arteries. The resection of calcified leaflets 1 should be done saving a portion of valve commissures 2 able to guarantee a stable anchoring of the prosthetic aortic valve at valve annulus level 9. The aortic annulus resection profile 5 and commissures 2 will be the part of the remaining aortic annulus anatomy in which the anchoring systems of the sutureless transcatheter valve prosthesis should be positioned.

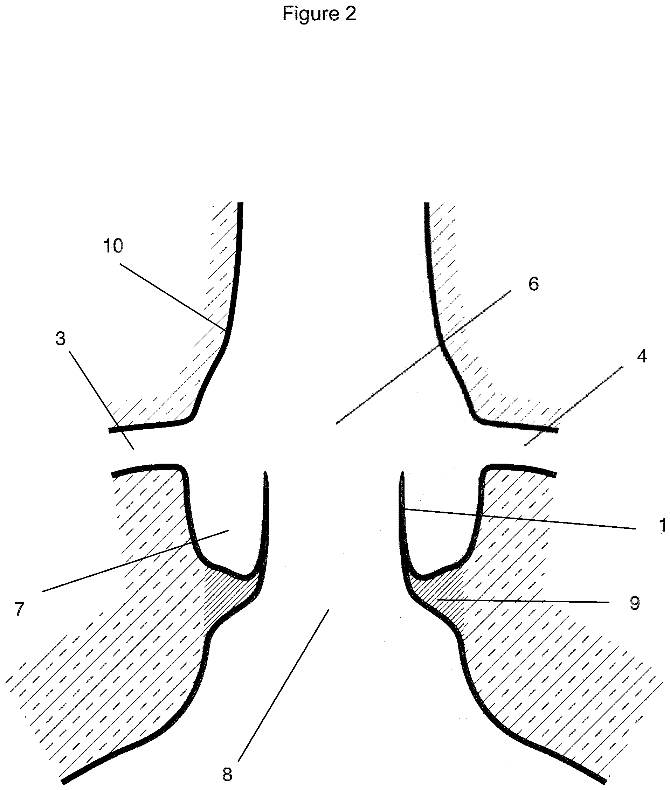

In Figure 2 a longitudinal section of the aorta is represented. In the figure the coronary sinuses 7 and coronary arteries 3,4 as well the aortic leaflets 1 are visible.

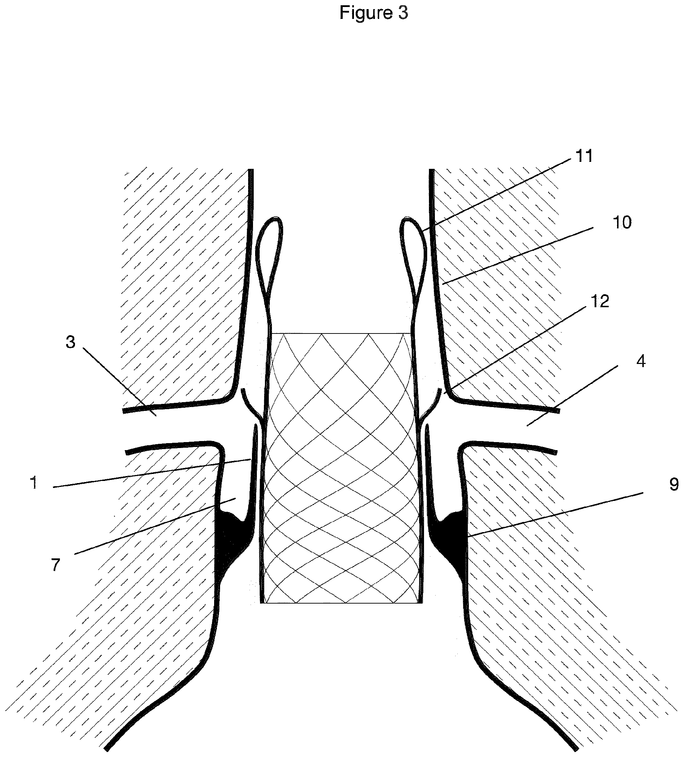

The positioning of a TAVI into a native stenotic aortic valve is described (Figure 3). In this representation, as example, a self-expandable prosthetic valve, currently in clinical use, is deployed. The anchoring of this valve prosthesis is mainly granted by the stenotic leaflets 1 of the native valve that are gripping on the stent structure at annulus level 9 and by the supra-annular anchoring flare 12 and the stabilization loops (sinusal anchoring structures 11) at sinotubular junction level 10. The supra-annular anchoring flare 12 are seating over the leaflets' 1 free margin with the scope preventing the ventricular migration of the prosthesis. On reverse, the sinusal anchoring structures 11, in contact with the aortic wall 10, reduce the prosthetic tilting movement that could favor its dislodgement into the aorta.

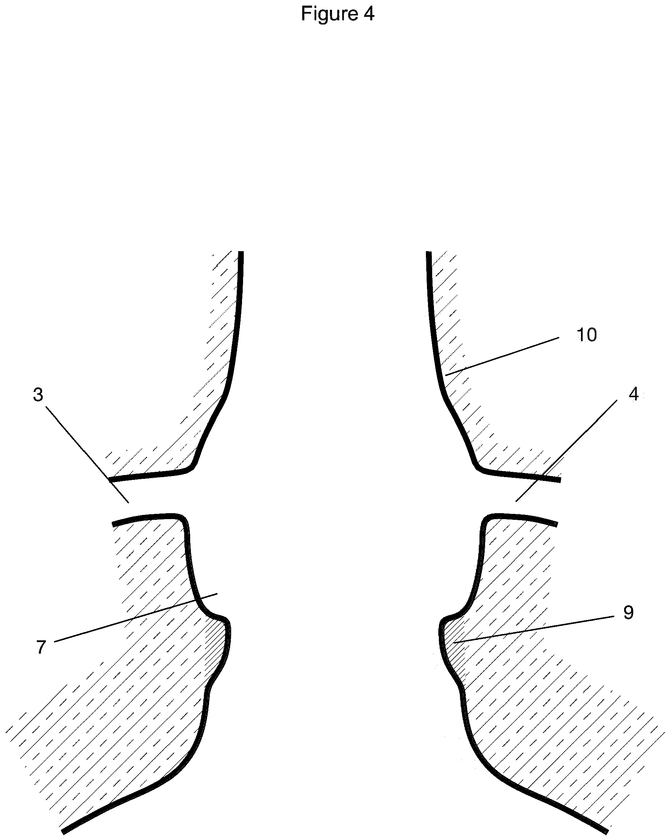

in Figure 4 a longitudinal section of the aorta is represented. In the figure the coronary sinuses 7 and coronary arteries 3,4 are visible, while are not visible the aortic valve leaflets 1 because resected.

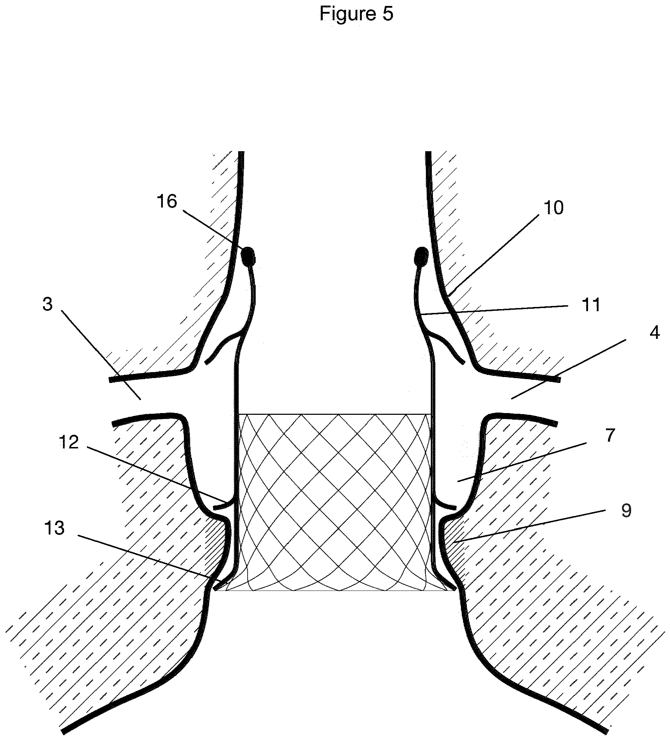

In Figure 5 the positioning of a sutureless prosthetic valve, into an annulus 9 with resected aortic native valve leaflets 1, is described. In this representation, as example, a self-expandable prosthetic valve is deployed. The anchoring of this valve prosthesis is mainly granted by three symmetrical level of anchoring.

The sub-annular anchoring flare 13 and the supra-annular anchoring flare 12 of the stent structure are perfectly cinching the resected annulus 9 preventing dislodgement and leakages.

The sinusal anchoring structures 11 in contact with the aortic wall 10, reduces the prosthetic tilting movement that could favor its dislodgement into the aorta. The sinusal structures include radiopaque markers 16 used during valve deployment. Additional radiopaque markers can be positioned at level of sub-annular anchoring flare 13, supra-annular anchoring flare 12 anchoring as well as at commissural level with the aim to obtain a safe and effective valve deployment and alignment with the coronary sinuses.

In Figure 6 is represented a flat design of a self-expandable stent structure. The bottom portion of the stent is conceived with a sub-annular asymmetrical anchoring flare 13 to prevent atrial-ventricular conduction blockage (on left side close to mitro-aortic continuity. The middle portion of the stent has a supra-annular anchoring flare 12 to prevent leakages and a sinusal anchoring structures 11 used to reduce the prosthetic tilting movement. Radiopaque markers 16, present on sinusal anchoring structures, are used during valve deployment to align it within the native valve commisures.

In Figure 7, a 3D configuration of the self-expandable stent represented in Figure 6. Visible the asymmetrical flared sub-annular anchoring 13, the supra-annular anchoring flare 12 to get in contact with the aortic annulus (cinching ridges that are formed after the full excision of said native valve leaflets) and the sinusal anchoring structures 11 with radiopaque markers 16 used during deployment.

In Figure 8 is represented the assembled prosthetic valve as represented in Figures 7 deployed in the aortic root. Are perfectly visible the functional pericardial or polymeric tissue leaflets 14 and the external pericardial or polymeric tissue cover 15 used to prevent paravalvular leakages at annulus level 9. The self-expandable prosthetic valve is ensuring three level anchoring with asymmetrical sub-annular anchoring flare 13, supra-annular anchoring flare 12 and sinusal anchoring structures 11 with radiopaque markers 16.

In Figure 9 is represented a flat design of a self-expandable stent structure. The bottom portion of the stent is conceived with a sub-annular symmetrical anchoring flare 13. The middle portion of the stent has a supra-annular anchoring flare 12 to prevent leakages and a sinusal anchoring structures 11 used to reduce the prosthetic tilting movement. Radiopaque markers 16 are visible and used during deployment of the stent.

In Figure 10, a 3D configuration of the self-expandable stent represented in Figure 9. Visible the asymmetrical flared sub-annular anchoring flare 13, the supra-annular anchoring flare 12 to get in contact with the aortic annulus (cinching ridges that are formed after the full excision of said native valve leaflets) and the sinusal anchoring structures 11 with radiopaque markers 16 used during deployment.

In Figure 11 is represented the assembled prosthetic valve as represented in Figures 10 deployed in the aortic root. Are perfectly visible the functional pericardial or polymeric tissue leaflets 14 and the external pericardial or polymeric tissue cover 15 used to prevent paravalvular leakages at annulus level 9. The self-expandable prosthetic valve is ensuring three level anchoring with asymmetrical sub-annular anchoring flare 13, supra-annular anchoring flare 12 and sinusal anchoring structures 11 with radiopaque markers 16. While the bottom part of the prosthesis (sub-annular anchoring flare 13) is covered by pericardial or polymeric tissue 15 to prevent paravalvular leakages, the upper part of the prosthesis (sinusal anchoring structures 11) is not covered by any pericardial or polymeric tissue to minimize the risk of coronary arteries obstruction.

In Figure 12 is represented another flat design of a self-expandable stent structure. The bottom portion of the stent is conceived with a sub-annular symmetrical anchoring flare 13. The middle portion of the stent has a supra-annular anchoring flare 12 to prevent leakages and a sinusal anchoring structures 11 used to reduce the prosthetic tilting movement. Radiopaque markers 16 are visible and used during deployment of the stent.

In Figure 13 a 3D configuration of the self-expandable stent represented in Figure 12. Visible the symmetrical flared sub-annular anchoring flare 13, the supra-annular anchoring flare 12 to get in contact with the aortic annulus (cinching ridges that are formed after the full excision of said native valve leaflets) and the sinusal anchoring structures 11 with radiopaque markers 16 used during deployment.

In Figure 14 is represented the assembled prosthetic valve as represented in Figures 13 deployed in the aortic root. Are perfectly visible the functional pericardial or polymeric tissue leaflets 14 and the external pericardial or polymeric tissue cover 15 used to prevent paravalvular leakages at annulus level 9. The self-expandable prosthetic valve is ensuring three level anchoring with symmetrical sub-annular anchoring flare 13, supra-annular anchoring flare 12 and sinusal anchoring structures 11 with radiopaque markers 16. While the bottom part of the prosthesis (sub-annular anchoring flare 13) is covered by pericardial or polymeric tissue 15 to prevent paravalvular leakages, the upper part of the prosthesis (sinusal anchoring structures 11) is not covered by any pericardial or polymeric tissue to prevent coronary arteries obstruction. Sinusal anchoring structures 11 are going well in contact with the aorta wall at the level of sinotubular junction 10 reducing the prosthetic tilting movement that could favor its dislodgement into the aorta.

In Figure 15 is represented the same flat design of a self-expandable stent structure of Figure 12. The bottom portion of the stent is conceived with a sub-annular symmetrical anchoring flare 13. The middle portion of the stent has a supra-annular anchoring flare 12 to prevent leakages and a sinusal anchoring structures 11 used to reduce the prosthetic tilting movement. Radiopaque markers 16 are visible and used during deployment of the stent.

In Figure 16 another 3D configuration of the self-expandable stent represented in Figure 12 and 15. Visible the symmetrical sub-annular anchoring flare 13, the supra-annular anchoring flare 12 to get in contact with the aortic annulus (cinching ridges that are formed after the full excision of said native valve leaflets) and the sinusal anchoring structures 11 with radiopaque markers 16 used during deployment. Differently from 3D of configuration of Figure 13, this stent has more pronounced bent out structures used to anchor the stent into the anatomy. Specifically, regarding the supra-annular anchoring flare 12 is used another part of the stent structure while the sinusal anchoring structures 11 are more bent out to increase coaptation within the aortic wall.

In Figure 17 is represented the assembled prosthetic valve as represented in Figures 16 deployed in the aortic root. Are perfectly visible the functional pericardial or polymeric tissue leaflets 14 and the external pericardial or polymeric tissue cover 15 used to prevent paravalvular leakages mainly at annulus level 9. The self-expandable prosthetic valve is ensuring three level anchoring with symmetrical sub-annular anchoring flare 13, supra-annular anchoring flare 12 and sinusal anchoring structures 11 with radiopaque markers 16. While the bottom part of the prosthesis (sub-annular anchoring flare 13) is covered by pericardial or polymeric tissue 15 to prevent paravalvular leakages, the upper part of the prosthesis (sinusal anchoring structures 11) is not covered by any pericardial or polymeric tissue to prevent coronaries obstructions. Sinusal anchoring structures 11 are going well in contact with the aorta wall at the level of sinotubular junction 10 reducing the prosthetic tilting movement that could favor its dislodgement into the aorta. In Figure 18 is represented another embodiment of the assembled prosthetic valve as represented in Figures 16 deployed in the aortic root. Are visible the functional pericardial or polymeric tissue leaflets 14 and the external pericardial or polymeric tissue cover 15 used to prevent paravalvular leakages mainly at annulus level 9. The self-expandable prosthetic valve is ensuring three symmetrical level anchoring. In this configuration, while the sub-annular anchoring flare 13 is similar to figure 17, the supra-annular anchoring flare 12 and sinusal anchoring structures 11 with radiopaque markers 16 are less bended out towards the aortic wall. While the bottom part of the prosthesis (sub-annular anchoring flare 13) is covered by pericardial or polymeric tissue 15 to prevent paravalvular leakages, the upper part of the prosthesis (sinusal anchoring structures 11) is not covered by any pericardial or polymeric tissue to prevent coronaries obstructions. Sinusal anchoring structures 11 are going well in contact with the aorta wall at the level of sinotubular junction 10 reducing the prosthetic tilting movement that could favor its dislodgement into the aorta.

In Figure 19 is represented another embodiment of the assembled prosthetic valve as represented in Figures 16 deployed in the aortic root. Are visible the functional pericardial or polymeric tissue leaflets 14 and the external pericardial or polymeric tissue cover 15 used to prevent paravalvular leakages mainly at annulus level 9. The self-expandable prosthetic valve is ensuring three symmetrical level anchoring. In this configuration the sub-annular anchoring flare 13 is less bended out respect Figure 18, while the supra-annular anchoring flare 12 and sinusal anchoring structures 11 with radiopaque markers 16 are more bended out towards the aortic wall. While the bottom part of the prosthesis (sub-annular anchoring flare 13 and supra-annular anchoring flare 12) are covered by pericardial or polymeric tissue 15 to prevent paravalvular leakages, the upper part of the prosthesis (sinusal anchoring structures 11) is not covered by any pericardial or polymeric tissue to prevent coronaries obstructions. Sinusal anchoring structures 11 are going well in contact with the aorta wall at the level of sinotubular junction 10 reducing the prosthetic tilting movement that could favor its dislodgement into the aorta.

In Figure 20 is represented the flat design of a balloon-expandable stent structure. The balloon-expandable prosthetic valve is ensuring only two symmetrical level anchoring. The bottom portion of the stent is conceived with a sub-annular symmetrical anchoring flare 13 while the sinusal anchoring structures 11 is also bended out and used to reduce the prosthetic tilting movement. No supra-annular anchoring flare 12 is foreseen for the balloon-expandable stent.

In Figure 21 the 3D configuration of the balloon-expandable stent represented in Figure 20. Visible the symmetrical sub-annular anchoring flare 13 and the sinusal anchoring structures 11. It's also visible the stent structure to sustain leaflet commissure stitches 17.

In Figure 22 is represented the assembled prosthetic valve as represented in Figures 21 deployed in the aortic root. Are visible the functional pericardial or polymeric tissue leaflets 14 and the external pericardial or polymeric tissue cover 15 used to prevent paravalvular leakages mainly at annulus level 9. This balloon-expandable prosthetic valve is ensuring two levels anchoring with symmetrical sub-annular anchoring flare 13 and sinusal anchoring structures 11. While the bottom part of the prosthesis (sub-annular anchoring flare 13 level) is covered by pericardial or polymeric tissue 15 to prevent paravalvular leakages, the upper part of the prosthesis (sinusal anchoring structures 11 level) is not covered by any pericardial or polymeric tissue to prevent coronaries obstructions. Sinusal anchoring structures 11 are going well in contact with the aorta wall at the level of sinotubular junction 10 reducing the prosthetic tilting movement that could favor its dislodgement into the aorta.

In Figure 23 is represented the flat design of a balloon-expandable stent structure. The balloon-expandable prosthetic valve is ensuring three symmetrical levels of anchoring with a sub-annular symmetrical anchoring flare 13, the supra-annular symmetrical anchoring flare 12 and the intra-sinusal anchoring structures 11 bended out and used to reduce the prosthetic tilting movement. To ensure a better grip of the valve within the anatomy, the balloon is mostly inflated at sinotubular junction 10 level. It's also visible the stent structure to sustain leaflet commissure stitches 17.

In Figure 24 the 3D configuration of the balloon-expandable stent represented in Figure 23. Visible the symmetrical sub-annular anchoring flare 13, supra-annular anchoring flare 12 and the intra-sinusal anchoring structures 11. It's also visible the stent structure to sustain leaflet commissure stitches 17. Both sub-annular 13 and supra-annular anchoring flare 12 are used to cinch ridges that are formed after the full excision of said native valve leaflets.

In Figure 25 is represented the assembled prosthetic valve as represented in Figures 24 deployed in the aortic root. Are visible the functional pericardial or polymeric tissue leaflets 14 and the external pericardial or polymeric tissue cover 15 used to prevent paravalvular leakages mainly at annulus level 9. The balloon-expandable prosthetic valve is ensuring three level anchoring with symmetrical sub-annular anchoring flare 13, symmetrical supra-annular anchoring flare 12 and intra-sinusal anchoring structures 11. While the bottom part of the prosthesis (sub-annular anchoring flare level 13 and supra-annular anchoring flare level 12) is covered by pericardial or polymeric tissue 15 to prevent paravalvular leakages, the upper part of the prosthesis (sinusal anchoring structures 11 level) is not covered by any pericardial or polymeric tissue to prevent coronaries obstructions. Intra-sinusal anchoring structures 11 are going well in contact with the coronary sinuses 7 below the sinotubular junction level 10. In this embodiment the outflow portion of the valve prosthesis is extended into the aorta above the sinotubular junction 10 so that the intra-sinusal structures 11 are avoiding the migration of the prosthesis into the ventricle while the upper row of rhomboids prevents the tilting movement.

In Figure 26 is represented the flat design of a low-profile balloon-expandable stent structure. In this balloon-expandable prosthetic valve is ensuring only one level anchoring with a sub-annular symmetrical anchoring flare 13. It's also visible the stent structure to sustain leaflet commissure stitches 17.

In Figure 27 the 3D configuration of the balloon-expandable stent represented in Figure 26. Visible the only symmetrical sub-annular anchoring flare 13. It's also visible the stent structure to sustain leaflet commissure stitches 17.

In Figure 28 is represented the assembled prosthetic valve as represented in Figures 27 deployed in the aortic root. Is visible the external pericardial or polymeric tissue cover 15 used to prevent paravalvular leakages at annulus level 9. The low-profile balloon-expandable prosthetic valve is ensuring one level anchoring with symmetrical sub-annular anchoring flare 13. Due to the low profile, all the prosthesis is covered by pericardial or polymeric tissue 15 to prevent paravalvular leakages. The prosthesis is always well anchored to the anatomy thanks to the balloon dilation during the deployment. No stent structures are element of obstruction of the right 3 and left 4 coronaries.

In Figure 29 is represented the flat design of a low-profile balloon-expandable stent structure. In this balloon-expandable prosthetic valve is ensuring two levels anchoring with a sub-annular symmetrical anchoring flare 13 and supra-annular anchoring flare 12. It's also visible the stent structure to sustain leaflet commissure stitches 17.

In Figure 30 the 3D configuration of the balloon-expandable stent represented in Figure 29. Visible the symmetrical flared sub-annular anchoring 13 and the supra-annular anchoring flare 12. It's also visible the stent structure to sustain leaflet commissure stitches 17.

In Figure 31 is represented the assembled prosthetic valve as represented in Figures 30 deployed in the aortic root. Is visible the external pericardial or polymeric tissue cover 15 used to prevent paravalvular leakages at annulus level 9. The low-profile balloon-expandable prosthetic valve is ensuring two levels anchoring with symmetrical sub-annular anchoring flare 13 and supra-annular anchoring flare 12 ensuring the cinching of ridges that are formed after the full excision of said native valve leaflets. Due to the low profile, all the prosthesis is covered by pericardial or polymeric tissue 15 to prevent paravalvular leakages. The prosthesis is always well anchored to the anatomy thanks to the balloon dilation during the deployment. No stent structures are element of obstruction of the right 3 and left 4 coronaries.

In Figure 32 is represented a similar flat design of a low-profile balloon-expandable stent structure as represented in Figure 29. In this valve design a different stent structure to sustain leaflet commissure stitches 17 is shown. Both symmetrical sub-annular 13 and supra-annular anchoring flare 12 are used to cinch the ridges that are formed after the full excision of native valve leaflets and prevent paravalvular leakages at annulus level 9.

In Figure 33 the 3D configuration of the low-profile balloon-expandable stent represented in Figure 32. Visible the symmetrical flared sub-annular anchoring 13 and the supra-annular anchoring flare 12. In this configuration both sub-annular 13 and supra-annular anchoring flare 12 are more bended out to ensure a better grip at annulus level. It's also visible the stent structure to sustain leaflet commissure stitches 17.

In Figure 34 is represented the assembled low-profile prosthetic valve as represented in Figures 33 deployed in the aortic root. Is visible the external pericardial or polymeric tissue cover 15 used to prevent paravalvular leakages at annulus level 9. The low-profile balloon-expandable prosthetic valve is ensuring two level anchoring with symmetrical sub-annular anchoring flare 13 and supra-annular anchoring flare 12 ensuring the cinching of the ridges that are formed after the full excision of native valve leaflets. Due to the low-profile, all the prosthesis is covered by pericardial or polymeric tissue 15 to prevent paravalvular leakages. The prosthesis is always well anchored to the anatomy thanks to the balloon dilation during the deployment. No stent structures are element of obstruction of the right 3 and left 4 coronaries.

In Figure 35a and 35b a special version of double balloon used for two step prosthetic valve (Figure 23, 24, 25) deployment into the aortic root is represented. Is Figure 35a is visible the inflated stent expansion balloon 18 inside the valve that is granting a full valve opening and the deflated balloon 19 for the sinusal stent level expansion. In Figure 35b is visible the second step of valve deployment, where the expansion balloon 19 for sinusal stent structures is inflated. The three bulge design of the balloon is granting a perfect expansion of the stent (at sinusal anchoring structures 11 level) at coronary sinuses level 7.

In Figure 36a and 36b a special version of a triple balloon used for three steps prosthetic valve deployment (Figure 20, 21, 22) into the aortic root is represented. Is Figure 36a the inflated stent expansion balloon 18 inside the valve, that is granting a full valve deployment, is visible. The balloons 19 and 20 are still deflated. In Figure 36b the two special tubular design balloons 19 and 20 are inflated respectively granting a perfect expansion of the sinusal anchoring structures 11 the sub-annular anchoring flare 13.

## Claims

1. Sutureless transcatheter heart valve prosthesis comprising a collapsible stent structure with anchoring elements and a valve component located within said stent structure; **characterized by the fact** that the stent structure is configured to be positioned within a heart valve annulus after full excision of the native valve leaflets (1); the said stent structure comprising a sub-annular anchoring element (13), a supra-annular anchoring element (12), and wherein said sub- and supra-annular anchoring elements (13,12)are configured to cinch ridges (9) that are formed by the valve commissures (2) and the resection profile (5) after the full excision of said native valve leaflets (1).

2. Heart valve prosthesis according to claim 1 wherein said prosthesis is an aortic valve prosthesis and wherein the stent structure is configured to be positioned within an aortic root; the said stent structure furthermore comprising a sinusal anchoring element (11).

3. Heart valve prosthesis according to claim 1 wherein said prosthesis is a pulmonary, a mitral or a tricuspid valve prosthesis.

4. Heart valve prosthesis according to anyone of the previous claims **characterized by** an inflow or proximal ring and an outflow or distal ring joined by at least two vertical structures or commissural pillars devoted to sustaining the functional component of the prosthesis.

5. Heart valve prosthesis according to anyone of the previous claims wherein the sub-annular anchoring element (13) and the supra-annular anchoring element (12), when fixed against the valve annulus, are oriented in opposite directions in a way that is allowing the recapturing of the prosthesis in case of incorrect deployment.

6. Heart valve prosthesis according to anyone of the previous claims wherein said anchoring elements (13,12,11) have a petal or a flange shape.
